# EUROPEAN PATENT APPLICATION

(11) **EP 3 632 927 A1**
(43) Date of publication of application: **08.04.2020**
(21) Application number: 17912123.1
(22) Date of filing: 31.05.2017
(51) Int. Cl.: C07K 14/47

(54) **KERATIN DERIVATIVE OBTAINABLE AT HIGH YIELD, AND METHOD OF USING SAME**

(71) Applicant: Little Scientist Co., Ltd., Ichinomiya-shi, Aichi 494-0001 (JP)
(72) Inventor: NOMURA, Yasutoshi, Ichinomiya-shi Aichi 494-0001 (JP); TSUBOI, Takayuki, Ichinomiya-shi Aichi 494-0001 (JP)
(74) Representative: Office Freylinger
(86) International application number: PCT/JP2017/020230
(87) International publication number: WO 2018/220739

(57) **Abstract**

Provided are a keratin derivative that is obtainable at very high yield and has low denatured protein content, and a method of using the same. The keratin derivative of the present invention is obtained, without using excessive chemical agents, from hair from which the surface cuticle, which is resistant to dissolution, decomposition, and conversion to derivatives, has been partially or entirely removed. In particular, aminoethyl disulfide keratin produced by easily converting some or all of disulfide bonds (-S-S-) into aminoethyl disulfide groups by aminoethanethiol is a desirable keratin derivative in terms of stability and solubility. In addition, the keratin derivative can be obtained at high yield, contains little denatured keratin, and can be inexpensively formed into artificial blood vessels, surgical sutures, wound dressing sheets, artificial tortoiseshell, artificial ivory, artificial leather, wool nanofibers, and the like.

## Description

### TECHNICAL FIELD

The present invention relates to a keratin derivative that is characterized by being produced from mammalian hair, such as wool or head hair, from which the cuticle has been partially or entirely removed, and to a method of using the keratin derivative, in which the keratin derivative is obtainable at high yield and industrially applicable with low costs.

Furthermore, the keratin derivative is characterized by being aminoethyl disulfide keratin produced by a step of immersing cuticle-removed hair in an aminoethanethiol-containing liquid to convert disulfide bonds in keratin into aminoethyl disulfide groups and a step of dissolving the hair containing keratin having aminoethyl disulfide bonds converted from disulfide bonds.

### BACKGROUND ART

Keratin, which is a protein contained in, for example, wool and head hair, is compatible with wool, feathers, silk, cotton, and head hair, and known to be effective in moisturizing fibers, such as head hair, and improving the sense of touch. Since keratin is a protein having many intramolecular or intermolecular disulfide bonds (-S-S-), keratin is known to form a structure with higher strength and more stability than other proteins. However, there is no method of efficiently extracting keratin from wool or head hair.

Keratin, a polymer containing many water-soluble amino acids, is insoluble in oily solvents. Keratin is also a protein having a molecular weight of tens of thousands. Keratin normally has intermolecular bonding through disulfide bonds to form a fibrous material having a very large molecular weight and is thus insoluble in water. For this, keratin has been made soluble in water by reducing disulfide bonds of keratin into thiol groups (-SH) or derivative groups thereof to cleave the disulfide bonds. For example, Patent Literature 1 describes a cosmetic formulation containing a peptide having -SS-(CH₂)ₙCOO- as a side chain group. Patent Literature 2 describes a water-soluble protein produced by reducing disulfide bonds of a water-insoluble protein into thiol groups and converting some or all of the thiol groups into carboxymethyl disulfide groups (-SSCH₂COOH).

However, in conversion into various derivatives to improve keratin solubility, cysteine residues forming disulfide bonds in the protein are oxidized and converted into cysteic acid to form a denatured protein. The conversion of disulfide bonds or thiol groups of cysteine residues into cysteic acid is caused by, for example, an oxidizing agent, heat, or an alkali. Although there are methods for converting into carboxymethyl disulfide groups and the like as described above in Patent Literature, these methods do not offer fundamental solutions for the irreversible conversion into cysteic acid.

Hair that is mammalian body hair, such as wool or human head hair, has a hard keratin layer called a cuticle on the outermost layer. The exposure of hair to excessive chemical agents and extreme reaction conditions for converting keratin contained in this keratin layer into a derivative produces a denatured protein. The denatured protein lacks disulfide bonds and loses keratin original characteristics and functions.

### CITATIONS LIST

### PATENT LITERATURES

Patent Literature 1: Japanese Patent Application Laid-Open No. 2010-285401
Patent Literature 2: Japanese Patent Application Laid-Open No. 7-126296

### SUMMARY OF INVENTION

### TECHNICAL PROBLEMS

Mammalian body hair, such as wool or head hair, has the surface coated with a hard keratin layer called a cuticle. The cuticle prevents hydrolysis or a chemical agent for conversion into a keratin derivative from permeating the inside of the cuticle and reduces hydrolysis or derivative conversion yield. There is thus a need to use excessive chemical agents and extreme reaction conditions for decomposition of a hard cuticle and conversion into a derivative, which causes generation of a large amount of a denatured protein.

An object of the present invention is to provide a keratin derivative that is obtainable at very high yield and has low denatured protein content, and a method of using the keratin derivative.

### SOLUTIONS TO PROBLEMS

The present invention provides a keratin derivative obtained, without using excessive chemical agents, from hair from which the surface cuticle, which is resistant to dissolution, decomposition, and conversion to derivatives, has been partially or entirely removed. In particular, aminoethyl disulfide keratin produced by easily converting some or all of disulfide bonds (-S-S-) into aminoethyl disulfide groups by aminoethanethiol is a desirable keratin derivative in terms of stability and solubility.

### ADVANTAGEOUS EFFECTS OF INVENTION

The keratin derivative of the present invention produced from hair from which the cuticle has been partially or entirely removed is obtainable at high yield without using excessive chemical agents and has low denatured keratin derivative content. In addition, the keratin derivative of the present invention contains little denatured keratin and can be inexpensively and stably formed into artificial blood vessels, surgical sutures, wound dressing sheets, artificial tortoiseshell, artificial ivory, artificial leather, wool nanofibers, and the like.

Furthermore, in aminoethyl disulfide keratin produced by a step of immersing hair in an aminoethanethiol-containing liquid to convert disulfide bonds in keratin into aminoethyl disulfide groups and a step of dissolving the hair containing keratin having aminoethyl disulfide bonds converted from disulfide bonds, the molecular weight of the keratin protein is not reduced, and the thiol groups of cysteine residues in the keratin that form disulfide bonds are protected to prevent denaturalization.

The disulfide bonds in keratin are intramolecular or intermolecular disulfide bonds between cysteine residues in keratin and are involved in keratin original strength and the maintenance and reconstruction of the keratin molecular structure.

By dissociating disulfide bonds in keratin into thiol groups, keratin insoluble in solvents can be made soluble in solvents. By forming disulfide bonds again, soluble keratin can be made into insoluble keratin. The dissociation and re-bonding of disulfide bonds are reversible, but denatured keratin in which cysteine is converted into cysteic acid loses the reversible reaction.

The aminoethyl disulfide keratin of the present invention can be produced without using excessive chemical agents and contains little denatured keratin. In addition, the cysteine residues of the keratin are protected in the form of aminoethyl disulfide groups and are resistant to conversion caused by, for example, oxygen in the air.

The keratin derivative of the present invention, especially aminoethyl disulfide keratin, can be formed into artificial blood vessels, surgical sutures, wound dressing sheets, artificial tortoiseshell, artificial ivory, artificial leather, wool nanofibers, and the like by controlling dissolution based on the dissociation and re-bonding of disulfide bonds in the keratin derivative.

### DESCRIPTION OF EMBODIMENT

The hair from which the cuticle has been partially or entirely removed is not particularly limited in terms of type and origin and should be any mammalian hair as long as the cuticle has been removed from the hair. Examples of the hair include sheep, angora, cashmere, mohair, camel, and alpaca hairs; and human head hair. A method of partially or entirely removing the cuticle is not particularly limited. The cuticle may be removed by a chemical treatment using chlorine or the like, or under a load using friction, a ball mill, or the like.

The keratin derivative may be obtained by converting disulfide bonds with use of, specifically, for example, thioglycolic acid, thiolactic acid, sulfurous acid, cysteine, spironolactone, salts of these sulfur-containing compounds, or derivatives thereof (e.g., esters) as long as the disulfide bonds in the keratin are stably protected without denaturalization. The sulfur-containing compounds may be used alone or in combination of two or more thereof.

The keratin derivative is particularly desirably aminoethyl disulfide keratin having an aminoethyl disulfide group in terms of stability and solubility.

The aminoethyl disulfide keratin of the present invention is produced by a step of immersing cuticle-removed hair in an aminoethanethiol-containing liquid to convert disulfide bonds in keratin into aminoethyl disulfide groups and a step of dissolving the hair containing keratin having aminoethyl disulfide bonds converted from disulfide bonds.

In the step of immersing hair in an aminoethanethiol-containing liquid, the amount of aminoethanethiol can be adjusted according to the amount of cysteine residues contained in the hair. Desirably, aminoethanethiol is added in an amount of 1.1 to 2 times the molar amount of keratin residues. The pH in the reaction is not particularly limited, but desirably in the range from pH 6 to 8.

In the dissolution, solvents such as alcohols, such as ethanol and propanol, and polyhydric alcohols, such as ethylene glycol and butylene glycol, or aqueous solutions may be used. The pH in the dissolution is not particularly limited, but desirably pH 6 or higher since the isoelectric point of the protein of the keratin derivative is about pH 3 to 5.

Some or all of disulfide bonds in keratin contained in the hair should be converted into aminoethyl disulfide bonds, and the step of immersing hair in an aminoethanethiol-containing liquid may be simultaneous with the step of the dissolution.

The conversion of disulfide bonds in keratin into aminoethyl disulfide groups by aminoethanethiol means that disulfide bonds are cleaved to form new bonds between S atoms and S atoms of aminoethanethiol (see formula (1) below).

Unconverted disulfide bonds may remain. If thiol groups formed by cleavage of disulfide bonds remain, a step of adding aminoethanethiol to all of the thiol groups by using an oxidizing agent is desirably added.

The oxidizing agent is not particularly limited in terms of type, origin, and structure as long as disulfide bonds can be formed between thiol groups. Specific examples of the oxidizing agent include hydrogen peroxide, bromic acid and salts thereof, chloric acid and salts thereof, ozone, and peroxide. The oxidizing agents may be used alone or in combination of two or more thereof.

Depending on the amount of aminoethanethiol and other reaction conditions, there are the case (formula (1) below) where one side of the disulfide bond is converted into an aminoethyl disulfide group and the other side is converted into a thiol group and the case (formula (2) below) where both sides of the disulfide group are converted into aminoethyl disulfide groups by aminoethanethiol. In the formulas (1) and (2), "Cys" means a cysteine residue contained in keratin.

Cys-S-S-Csy + H₂N-CH₂-CH₂-SH -> Cys-S-S-CH₂-CH₂-NH₂ + Cys-SH (1)

Cys-S-S-Csy + H₂N-CHR-CH₂-SH -> 2 Csy-S-S-CH₂-CHR-NH₂ (2)

The keratin derivative may be subjected to other treatments as needed. For example, the keratin derivative may be hydrolyzed in order to reduce the molecular weight and the molecular size. In addition, other components or insoluble matter may be removed by, for example, purification, filtration, or centrifugation.

The keratin derivative may contain other components as needed as long as the other components do not inhibit the keratin characteristics during use. Examples of the other components include fats and oils, hydrocarbon oils, higher fatty acids, higher alcohols, ester oils, silicone oils, surfactants (anionic, cationic, amphoteric, nonionic surfactants), moisturizers, water soluble polymers, film forming agents, UV absorbers, sequestering agents, lower alcohols, polyhydric alcohols, saccharides, amino acids, pH adjusters, vitamins, antioxidants, pigments, preservatives, and flavors.

The keratin derivative obtained from the hair from which the cuticle has been partially or entirely removed has undergone little conversion into cysteic acid because the keratin derivative is produced under moderate reaction conditions. The keratin derivative also has protected disulfide bonds and has good shape processability. In addition, the keratin derivative can be treated as a solution, and is easily formed into a nanofiber having a smaller fiber diameter than the original hair.

The disulfide bonds in the keratin derivative of the present invention can be cleaved or re-bonded. The re-bonding of the disulfide bonds in the formed state can stably maintain the formed state and enables formation of artificial blood vessels, surgical sutures, wound dressing sheets, artificial tortoiseshell, artificial ivory, artificial leather, artificial hair, wool nanofibers, and the like.

Any of the formed products needs to always maintain its formed shape, and the disulfide bonds, which are characteristics of a keratin protein, are important. Further, for example, aminoethyl disulfide groups, which protect cysteine residues of the keratin derivative, can be cleaved as needed to form thiols. The re-bonding of thiols formed by cleavage can regenerate original keratin.

The keratin derivative can be easily formed into the shapes of artificial blood vessels, surgical sutures, wound dressing sheets, artificial tortoiseshell, artificial ivory, artificial leather, wool nanofibers, and the like.

### EXAMPLES

The present invention will be specifically described below by way of Examples. The present invention is not limited to the modes illustrated in Examples. Various modifications of the embodiments of the present invention are possible according to purpose, application, or the like without departing from the scope of the present invention. All the discussions for the results of Examples are only inventors' views and do not provide description of the gist that defines the present invention.

### (Example 1)

### Method of preparing aminoethyl disulfide keratin from wool from which cuticle has been removed

Wool (1 kg) from which the cuticle has been removed is immersed in a pH-7.5 aqueous solution containing 1.2 molar equivalents of aminoethanethiol and left to stand at 40°C for one day. The wool is washed well with water and immersed in a liquid with pH 8.5 adjusted with sodium hydroxide. The liquid is stirred for two days to dissolve the wool. The pH required for using the keratin derivative is adjusted as needed. At this time, the yield of obtained aminoethyl disulfide keratin was 69.3%.

### (Comparative Example 1)

In the case of performing the same operation using cuticle-unprocessed wool, the yield of aminoethyl disulfide keratin was 13.1%. When the dissolution step was performed at a temperature of 60°C and pH 12, the yield was 21.7%, but the cysteine content in keratin was found to be obviously reduced. This indicates that cysteine residues are converted because the pH and the temperature are raised to dissolve hard cuticle in the dissolution step.

### (Example 2)

### Wound dressing sheet

Sodium hydrogen sulfite (5%) is added to a 10% aqueous solution of aminoethyl disulfide keratin to cause a reaction. At this time, aminoethyl disulfide groups are cleaved to form thiols of cysteine residues. Next, an aqueous solution containing the keratin derivative is placed in a tray, and a proper amount of hydrogen peroxide is added under alkaline conditions at pH 8 to form a keratin gel. By using hydrogen peroxide, disulfide bonds are formed between thiols of cysteine residues in keratin to cause crosslinking of keratin. The keratin gel is dried and washed well with sterilized water to provide a film-shaped wound dressing sheet. If a film formed only of keratin is hard and cannot be stuck so as to conform to a wound area at this time, an oil or fat, such as Vaseline, may be added to the initial aminoethyl disulfide keratin solution, or the keratin gel may be swelled with glycerol-containing water after dried.

### (Example 3)

### Nanofiber

Sodium hydrogen sulfite (5%) is added to a 10% aqueous solution of aminoethyl disulfide keratin to cause a reaction. Next, components other than polymer keratin are removed through a dialysis membrane, and hydrogen peroxide is then added under alkaline conditions at pH 8 using ammonia water to provide a keratin gel. The keratin gel is subjected to an electrospinning method or to an air spraying method in an environment equipped with a ventilation system to provide a nonwoven fabric formed of a keratin nanofiber. In particular, in the air spraying method, a roll-shaped nonwoven fabric can be produced easily, and the fiber diameter of the nanofiber can be controlled by controlling the spray pressure.

### (Example 4)

### Artificial ivory (artificial horn)

A 10% aqueous solution of aminoethyl disulfide keratin is adjusted to pH 8 by using sodium hydroxide, and 1% of cysteine is added to the solution to cause a reaction. Next, 2% of hydrogen peroxide is added to provide a keratin gel. The keratin gel is washed by using a dialysis membrane, and the keratin gel is dried and ground. A mold is filled with 30% of keratin powder and 70% of hydroxyapatite, and the mixture is subjected to heat-press molding at 150°C and a pressure of 600 kg/cm² for 10 minutes to form a prismatic or cylindrical artificial ivory. The hardness and texture can be changed by changing the mixing ratio, which makes it possible to produce artificial horns imitating buffalo horns or the like.

### (Example 5)

### Artificial tortoiseshell

A 10% aqueous solution of aminoethyl disulfide keratin is adjusted to pH 8 by using sodium hydroxide, and 1% of cysteine is added to the solution to cause a reaction. Next, 2% of hydrogen peroxide is added to provide a keratin gel. The keratin gel is washed by using a dialysis membrane, and polyvinyl alcohol is then added to the keratin gel. The keratin gel is dried to provide keratin sheets. Any number of keratin sheets are stacked on top of one another and hot-pressed at 90°C and a pressure of 100 kg/cm² for 5 minutes to provide artificial tortoiseshell.

### (Example 6)

### Biodegradable surgical suture

A 10% aqueous solution of aminoethyl disulfide keratin is adjusted to pH 8 by using sodium hydroxide, and 1% of cysteine is added to the solution to cause a reaction. Next, 2% of hydrogen peroxide is added to provide a keratin gel. The keratin gel is washed by using a dialysis membrane and subjected to replacement with sterilized water. Equal amounts of pig skin acid-treated gelatin (available from Nippi. Inc.) and 2% glutaraldehyde are then added to form a mixed solution, and the mixed solution is then ejected from a syringe at 4°C to form a thread. The thread is left to stand at 40°C for 24 hours, and the obtained thread is immersed in a 4 M glycine aqueous solution and further left to stand at 40°C for 24 hours. The thread is washed well with sterilized water to provide a biodegradable surgical suture. The biodegradable surgical suture becomes harder as the gelatin content decreases and becomes more flexible as the gelatin content increases.

### (Example 7)

### Artificial hair

A 10% aqueous solution of aminoethyl disulfide keratin is adjusted to pH 7 by using sodium hydroxide, and 2% of sodium hydrogen sulfite is added to the solution to cause a reaction. Next, 2% of hydrogen peroxide is added to provide a keratin gel. To the keratin gel, 30% of carbon black is added. The mixture is ejected from a syringe, dried, and washed with water, and dried again to provide artificial hair with natural texture.

### (Example 8)

### Artificial leather

A 5% aqueous solution of aminoethyl disulfide keratin is adjusted to pH 8 by using sodium hydroxide, and 1% of cysteine is added to the solution to cause a reaction. Next, 2% of hydrogen peroxide is added to provide a keratin gel. Low-molecular-weight components, such as hydrogen peroxide, are removed from the keratin gel by using a dialysis membrane, and a silk woven fabric is impregnated with the keratin gel and dried to provide artificial leather with texture similar to that of animal leather.

### (Example 9)

### Artificial blood vessel

A 10% aqueous solution of aminoethyl disulfide keratin is adjusted to pH 7 by using disodium hydrogen phosphate and sodium dihydrogen phosphate, and 2% of sodium hydrogen sulfite is added to the solution to cause a reaction. Next, 2% of hydrogen peroxide is added to provide a keratin gel. Excessive hydrogen peroxide is washed away by using a dialysis membrane to provide a keratin solution. Next, a tube, 3 mm in inner diameter, formed by knitting a polyethylene terephthalate fiber is immersed in the keratin solution and then dried. Keratin has disulfide bonds and can form a membrane with very high air-tightness, which prevents a liquid flowing through the inside of the tube from leaking to the outside of the tube. In addition, the tube when coated with a polyurethane resin or the like is expected to have a longer service life.

## Claims

1. A keratin derivative produced from mammalian hair, such as wool or head hair, from which a cuticle has been partially or entirely removed.

2. The keratin derivative according to claim 1, wherein the derivative is aminoethyl disulfide keratin produced by a step of immersing cuticle-removed hair in an aminoethanethiol-containing liquid to convert disulfide bonds in keratin into aminoethyl disulfide groups and a step of dissolving the hair containing keratin having aminoethyl disulfide bonds converted from disulfide bonds.

3. A method of using the keratin derivative according to claim 1 or 2, the method comprising forming the keratin derivative into an artificial blood vessel, a surgical suture, a wound dressing sheet, artificial tortoiseshell, artificial ivory, artificial leather, artificial hair, and a wool nanofiber.
